# EUROPEAN PATENT APPLICATION

(11) **EP 2 468 272 A1**
(43) Date of publication of application: **27.06.2012**
(21) Application number: 12151238.8
(22) Date of filing: 11.05.2007
(51) Int. Cl.: A61K 31/198, A61K 31/7004, A61P 25/00, A61P 25/14, A61P 25/16, A61P 25/28

(54) **Methods of treating a neurological disorder with creatine monohydrate**

(30) Priority: 11.05.2006 US 79974306 P; 06.04.2007 US 92214607 P
(62) Divisional of application: 07794769.5
(71) Applicant: Avicena Group, Inc., Boston MA 02199-8001 (US)
(72) Inventor: Nivaggioli, Belinda, Tsao, CA 94027 (US)
(74) Representative: HOFFMANN EITLE

(57) **Abstract**

The present invention provides methods of treating neurological disorders, such as Huntington's disease, Parkinson's disease and amyotrophic lateral sclerosis, by administering creatine monohydrate and dextrose, alone or in combination with an anti-inflammatory compound, to a subject.

## Description

### Related Applications

This application claims priority to U.S. Provisional Patent Application No. 60/922,146, filed on April 6, 2007; and U.S. Provisional Patent Application No. 60/799,743, filed on May 11, 2006. The aforementioned applications are hereby incorporated in their entirety by reference.

### Background of the Invention

Neurological disorders are disorders that affect the central nervous system, the peripheral nervous system or the autonomic nervous system. These neurological disorders include, for example, amyotrophic lateral sclerosis (ALS), Huntington's disease and Parkinson's disease. ALS, often referred to as "Lou Gehrig's disease," is a progressive neurodegenerative disease that attacks the motor neurons of the brain and spinal cord that are responsible for voluntary muscle movement. As these motor neurons degenerate their ability to send impulses to the muscle fibers is compromised. As the disease progresses, the motor neurons die, which results in the brain's inability to initiate or control muscle movement and, eventually, the patient becomes completely paralyzed and their muscles atrophy. ALS affects roughly 30,000 Americans at one time and every year 5600 new cases of ALS are diagnosed.

Huntington's disease is a progressive neurodegenerative disease caused by a genetic defect. The disease causes the deterioration of neurons in those parts of the brain that are responsible for controlling cognitive, emotional and motor functions. As a result, patients suffer a variety of symptoms including uncontrollable muscle movements, clumsiness, memory loss, and, ultimately, severe mental deterioration. In the United States, approximately 35,000 people suffer from Huntington's disease and another 175,000 people are at risk for developing the disease.

Parkinson's disease is a progressive, neurodegenerative brain disorder that occurs when neurons within the brain that are responsible for producing the chemical dopamine become impaired or die. The cause of this nerve cell damage and death is not completely understood. Eventually, symptoms, which include uncontrolled shaking of the hands and or feet, may progress to a point where routine tasks become severely impaired. It is estimated that approximately 1.5 million Americans are affected by Parkinson's disease, making it the second most common neurodegenerative disease after Alzheimer's disease. Approximately 60,000 new cases are diagnosed each year in the United States.

There are currently no known cures for ALS, Huntington's disease, Parkinson's disease and many other neurological disorders. Instead, treatment is focused on relieving symptoms, preventing complications and maximizing the quality of life.

### Summary of the Invention

The invention pertains, at least in part, to a method treating a neurological disorder in a human by administering to the human a composition comprising creatine monohydrate and dextrose. Examples of neurological disorders include, but are not limited to, Parkinson's disease, Alzheimer's disease, Huntington's disease, Charcot-Marie-Tooth disease, muscular dystrophy and amyotrophic lateral sclerosis.

The present invention also pertains, at least in part, to a method of treating a neurological disorder in a human by administering a composition comprising creatine monohydrate and dextrose in combination with an anti-inflammatory compound, such that the neurological disorder in said human is treated.

The present invention further pertains, at least in part, to a pharmaceutical composition comprising creatine monohydrate, dextrose and an anti-inflammatory compound.

The invention also pertains, at least in part, to a kit comprising a container of creatine monohydrate, dextrose and an anti-inflammatory compound and instructions for administering the creatine monohydrate, dextrose and an anti-inflammatory compound to a human such that the human is treated for a neurological disorder.

In another embodiment, the invention pertains, at least in part, to a packaged pharmaceutical composition for treatment of Parkinson's disease in which the composition comprises about 5 grams of creatine monohydrate and about 2 grams of dextrose and instructions for treating Parkinson's disease. In one embodiment, the instructions provide instructions for at least a twice daily administration.

In yet another embodiment, the invention pertains, at least in part, to a packaged pharmaceutical composition for treatment of amyotrophic lateral sclerosis in which the composition comprises about 5 grams of creatine monohydrate and about 2 grams of dextrose and instructions for treating amyotrophic lateral sclerosis. In one embodiment, the instructions provide instructions for at least a once daily administration.

The invention also pertains, at least in part, to a packaged pharmaceutical composition for treatment of Huntington's disease, in which the composition comprises about 15 grams of creatine monohydrate and about 2 grams of dextrose and instructions for treating Huntington's disease. In one embodiment, the instructions provide instructions for at least a twice daily administration.

### Brief Description of the Drawing

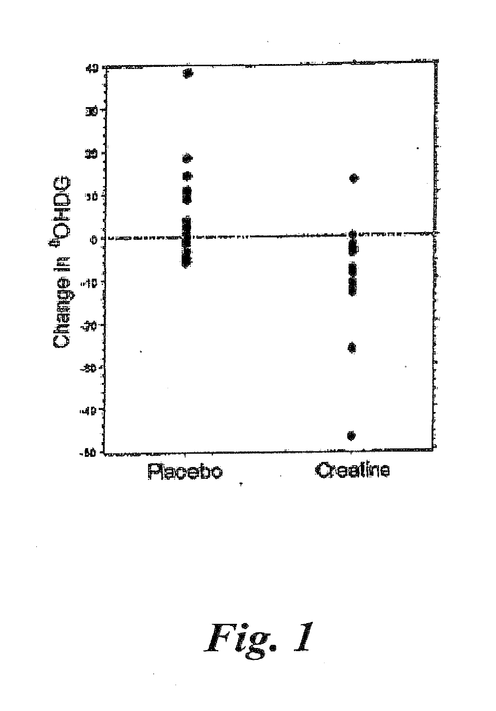

Figure 1 is a scatterplot that illustrates individual changes in 8-hydroxy-2'-deoxyguanosine (80H2'dG) (pg/mL) levels at week 16 in 32 placebo-treated subjects and 32 creatine-treated subjects.

### Detailed Description of the Invention

The present invention pertains, at least in part, to a method treating a neurological disorder in a human by administering to the human a composition comprising creatine monohydrate and dextrose.

The term "neurological disorder" refers to disorders that may cause a disturbance in the structure or function of the nervous system resulting from developmental abnormalities, disease, genetic defects, injury or toxin. These disorders may affect the central nervous system (*e.g*., the brain, brainstem and cerebellum), the peripheral nervous system (*e.g*., the cranial nerves, spinal nerves, and sympathetic and parasympathetic nervous systems) and/or the autonomic nervous system (*e.g.,* the part of the nervous system that regulates involuntary action and that is divided into the sympathetic and parasympathetic nervous systems). Examples of neurological disorders may include, for example, Landau-Kleffner syndrome, acquired epileptiform aphasia, acute disseminated encephalomyelitis, adrenoleukodystrophy, neurological complications of acquired immunodeficiency syndrome (AIDS), Alexander disease, Aiper's disease, amyotrophic lateral sclerosis, ataxia, ataxia-tefangiectasia, dysautonomia, autonomic dysfunction, familial dysautonomia, Riley-Day syndrome, benign essential blepharospasm, blepharospasm, monomelic amyotrophy, benign focal amyotrophy, Hirayama syndrome, O'Sullivan-McLeod syndrome, subcortical arteriosclerotic encephalopathy, traumatic brain injury, Brown-Sequard syndrome, Kennedy's disease, bulbospinal muscular atrophy, spinal muscular atrophy, Caravan disease, leukodystrophy, central cord syndrome, cerebellar degeneration, cerebral atrophy, Charcot-Marie-Tooth disease, chorea, dyskinesia, Syndenham chorea, neuroacantheytosis, Levine-Critchley syndrome, choreoacanthocytosis, chronic inflammatory demyelinating polyneuropathy (CIDP), congenital myasthenia, congenital myopathy, central core disease, nemaline rod myopathy, centronuclear (myotubular) myopathy, corticobasal degeneration, Creutzfeldt-Jakob disease, dementia, dyssenergia cerebellaris myoclonica, dyssenergia cerebellaris progressive, dentate cerebellar ataxia, dentatorubral atrophy, primary dentatum atrophy, Ramsey-Hunt syndrome, dermatomytosis, Devic's syndrome, Schilder's disease, myelinociastic diffuse sclerosis, dystonias, familial periodic paralysis, Friedreich's ataxia, Germann-Straussler-Scheinker disease, Krabbe disease, Guillain-Barre syndrome, hemiplegia alterans, tropical spastic paraparesis, retrovirus-associated myetopathy, HTLV-1 associated myelopathy, Huntington's disease, hypertonia, Isaac's syndrome, neuromyotonia, kuru, opsoclonus myoclonus, Kinsbourne syndrome, spinal muscular atrophy, Werdnig-Hoffman disease, Kugelberg-Welander disease, transmissible spongiform encephalopathies, fatal familial insomnia, Lambert-Eaton myasthenic syndrome, Leigh's disease, locked-in syndrome, Lou Gehrig's disease, lupus, systemic lupus erythematosus, Machado-Joseph disease, Melkersson-Rosenthal syndrome, Miller Fisher syndrome, mitochondrial myopathies, motor neuron disease, multifocal motor neuropathy, multiple sclerosis, multiple system atrophy, muscular dystrophy, myasthenia gravis, neurofibromatotis, von Recklinghausen's disease, neurological complications of Lyme's disease, thyrotoxic myopathy, tabes dorsalis, progressive locomotor ataxia, prion diseases, primary lateral sclerosis, acute demyelinating neuropathy, acute disseminated encephalomyelitis, acute necrotizing hemorrhagic leukoencephalitis, metachromic leukodystrophy, adrenoleukodystrophy; adrenomyeloneuropathy, spinocerebellar degenerations, mitochondrial encephalomyopathies, Pelizaeus-Merzbacher disease, creatine transporter defect, and Duchenne muscular dystrophy.

The language "treating a neurological disorder" is intended to include prevention of the disorder, amelioration and/or arrest of a preexisting disorder, and the elimination of a preexisting disorder, or the prevention, amelioration and/or arrest of one or more symptoms of the neurological disorder.

In one embodiment, compositions of the invention slow or prevent cortical thinning of the brain. The term "cortical thinning" refers to the decrease in the mass, thickness and/or surface area of the brain.

In another embodiment, the compositions of the invention modulate the levels of one or more biomarkers in the subject. The term "biomarker" includes any molecular species found to provide correlation to a particular phenotype or perturbation of a biological system and is used to indicate or measure a biological process (*e.g*., a neurological disorder). In one embodiment, the biomarker is serum 8-hydroxy-2'-deoxyguanosine (8OH2'dG). In a further embodiment, the level of 8OH2'dG in a subject suffering from or at risk of suffering from a neurological disorder is reduced to a level comparable to the level of 8OH2'dG in a healthy subject.

In yet another embodiment, the compositions of the invention modulate the levels of one or more genetic markers in said subject. The term "genetic marker" includes known DNA sequences that can be identified by a simple assay, for example, a short DNA sequence, such as a sequence surrounding a single base-pair change (single nucleotide polymorphism), or a long one DNA sequence, such as a microsatellite. Genetic markers can be used to study the relationship between a disease and its genetic cause (for example, a particular mutation of a gene that results in a defective protein). In an embodiment, the genetic marker is pleopmorphic adenoma gene-like 1 (PLAG1) or H2A histone family, member Y (H2AFY).

The term "modulate" includes, for example, the increase or decrease of the concentration of biomarker or the genetic marker found in a subject suffering from or at risk of suffering from a neurological disorder.

The composition of the present invention includes creatine monohydrate (also known as N-(aminoiminomethyl)-N-methylglycine monohydrate; methylglycosamine monohydrate or N-methyl-guanido acetic acid monohydrate) and dextrose (also known as α-D-glucose). Creatine monohydrate has the chemical formula:

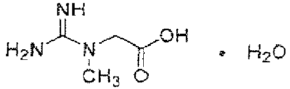

In a further embodiment, the creatine monohydrate of the invention is pharmaceutical grade creatine monohydrate. The term "pharmaceutical grade creatine monohydrate" includes compositions which are substantially free of toxins, such as, but not limited to, creatinine, dihydrotriazine, dicyandiamide, and heavy metals.

Pharmaceutical grade creatine and/or creatine monohydrate is substantially different from the creatine generally available on retail shelves for use as a nutritional supplement. Pharmaceutical grade creatine and creatine monohydrate is manufactured under drug GMP guidelines and meets the standards. Unlike nutritional supplement creatine, pharmaceutical grade creatine and creatine monohydrate is substantially free of neurotoxins or other toxins, which may be dangerous to subjects with neurodegenerative diseases (e,g. Report of the Scientific Committee on Food (SCF), Opinion on safety aspects of creatine supplementation (adopted by the SCF on 7 September 2000).

The term "substantially free of toxins" includes compositions with such low levels of toxins, (e.g., creatinine, dicyandiamide, dihydrotriazine, and heavy metals), that the composition is appropriate for administration to subjects with neurodegenerative diseases. The levels of toxins can be determined using methods known in the art such as chromatography, e.g., thin layer chromatography. In a further embodiment, the term "substantially free of toxins" includes compositions with less than about 200 ppm, less than about 100 ppm, or less than about 50 ppm of any individual toxin and/or less than about 1000 ppm, less than about 500 ppm, less than about 200 ppm, or less than about 100 ppm of total toxins in the creatine and/or creatine monohydrate,

in contrast to the pharmaceutical grade creatine claimed herein, nutriceutical grade creatine may contain heavy metals such as mercury and lead; about 54,000 ppm (5.4 %) dicyandiamide; about 13,000 ppm (1.3%) creatinine; and about 860 ppm dihydroazines.

In one embodiment, the pharmaceutical grade creatine monohydrate contains less than about 1% heavy metals, less than about 0.5%, less than about 0.1%, less than about 0.01%, less than about 0.001%, or substantially no heavy metals, such as lead and mercury.

In another further embodiment, the pharmaceutical grade creatine monohydrate contains less than about 24,000 ppm, less than about 20,000 ppm, less than about 10,000 ppm, less than about 5,000 ppm, less than about 1,000 ppm, less than about 500 ppm, less than about 100 ppm, less than about 70 ppm, or less than the detectable threshold of dicyandiamide.

In another further embodiment, the pharmaceutical grade creatine monohydrate contains less than about 13,000 ppm, less than about 10,000 ppm, less than about 8,000 ppm, less than about 6,000 ppm, less than about 4,000 ppm, less than about 2,000 ppm, less than about 1,000 ppm, less than about 500 ppm, less than about 100 ppm, less than about 50 ppm, less than about 20 ppm, or less than the detectable threshold of creatinine.

In another further embodiment, the pharmaceutical grade creatine monohydrate contains less than about 860 ppm, less than about 600 ppm, less than about 400 ppm, less than about 200 ppm, less than about 100 ppm, less than about 50 ppm, or less than the detectable threshold of dihydrotriazines.

In one embodiment, the amount of creatine monohydrate in the composition is between about 1 gram and about 50 grams. The term "about," as used with reference to an amount of creatine monohydrate and/or an anti-inflammatory compound and/or dextrose refers to ± 0.5 grams of creatine monohydrate and/or an anti-inflammatory compound and/or dextrose. In another embodiment, the amount of creatine monohydrate in the composition is about 1 gram, about 2 grams, about 3 grams, about 4 grams, about 5 grams, about 6 grams, about 7 grams, about 8 grams, about 9, grams, about 10 grams, about 11 grams, about 12 grams, about 13 grams, about 14 grams, about 15 grams, about 16, grams, about 17 grams, about 18 grams, about 19 grams, about 20 grams, about 21 grams, about 22 grams, about 23 grams, about 24 grams, about 25 grams, about 26 grams, about 27 grams, about 28 grams, about 29 grams, about 30 grams, about 31 grams, about 32 grams, about 33 grams, about 34 grams, about 35 grams, about 36 grams, about 37 grams, about 38 grams, about 39 grams, about 40 grams, about 41 grams, about 42 grams, about 43 grams, about 44 grams, about 45 grams, about 46 grams, about 47 grams, about 48 grams, about 49 grams or about 50 grams or greater. In another embodiment, the amount of creatine monohydrate is a therapeutically effective amount of creatine monohydrate.

In one embodiment, the amount of dextrose in the composition is between about I gram and about 20 grams. In another embodiment, the amount of dextrose in the composition is about 1 gram, about 2 grams, about 3 grams, about 4 grams, about 5 grams, about 6 grams, about 7 grams, about 8 grams, about 9, grams, about 10 grams, about 11 grams, about 12 grams, about 13 grams, about 14 grams, about 15 grams, about 16, grams, about 17 grams, about 18 grams, about 19 grams and about 20 grams, In another embodiment, the amount of dextrose is necessary to enhance the flow characteristics of the composition.

In one embodiment, the particle size of the creatine monohydrate and/or the dextrose is between about 1000 and 1500 microns, between about 100 and 1200 microns or about 1190 microns. The particle size of the creatine monohydrate and the dextrose is not particularly limited provided that the particle size does not affect the intended function of the composition (*e.g*., treating a neurological disorder). For example, in one embodiment, the particle size of the creatine monohydrate and the dextrose is the same.

The creatine monohydrate may also be mixed with any appropriate carrier. Suitable carriers include any pharmaceutically acceptable carrier (*e.g*., dextrose). An appropriate carrier can be selected such that it blends well with creatine monohydrate (*e.g*., similar size, consistency or color). The carrier may also be chosen to enhance the flow characteristics of creatine monohydrate.

In another embodiment, the invention pertains, at least in part, to a method of treating Parkinson's disease in a human by administering to the human a composition comprising creatine monohydrate and dextrose. In one embodiment, the method pertains to treating Parkinson's disease in a human by administering to the human a composition comprising between about 1 gram and about 50 grams of creatine monohydrate (*e.g*., about 5 grams) and between about 1 gram and 20 grams of dextrose (*e.g*., about 2 grams). The composition may be administered at least once a day (*e.g*., twice a day, three times a day, four times a day). The composition may be administered to the subject for treatment of Parkinson's disease, for example, in the amount of 5 grams of creatine monohydrate and 2 grams of dextrose at least twice a day.

In yet another embodiment, the invention pertains, at least in part, to a method of treating amyotrophic lateral sclerosis in a human by administering to the human a composition comprising creatine monohydrate and dextrose. In one embodiment, the method pertains to treating amyotrophic lateral sclerosis in a human by administering to the human a comprising between about 1. gram and about 50 grams of creatine monohydrate (*e.g*., about 5 grams) and between about 1 gram and 20 grams of dextrose (*e.g*., about 2 grams). The composition may be administered to the subject for treatment of amyotrophic lateral sclerosis, for example, in the amount of 5 grams of creatine monohydrate and 2 grams of dextrose at least once a day.

In another embodiment, the invention pertains, at least in part, to a method of treating Huntington's disease in a human by administering to the human a composition comprising creatine monohydrate and dextrose. In one embodiment, the method pertains to treating Huntington's disease comprising between about 1 gram and about 50 grams of creatine monohydrate (*e.g*., about 15 grams) and between about 1 gram and 20 grams of dextrose (*e.g*., about 2 grams). The composition may be administered to the subject for treatment of Huntington's disease, for example, in the amount of 15 grams of creatine monohydrate and 2 grams of dextrose at least twice a day.

The present invention also pertains to a method of treating a neurological disorder in a subject comprising administering to said subject a composition comprising creatine monohydrate, dextrose and an anti-inflammatory compound. In one embodiment, the neurological disorder is Huntington's disease, Parkinson's disease or amyotrophic lateral sclerosis. In another embodiment, invention also pertains to a method of treating a neurological disorder in a subject comprising administering to said subject a composition comprising between about 1 grams and about 50 grams creatine monohydrate (*e.g*., about 5 grams or about 15 grams), between about grams and about 20 grams dextrose (e.g., about 2 grams) and an anti-inflammatory compound.

The term "anti-inflammatory compound" refers compounds that treat, prevent or ameliorate inflammation in a subject. The anti-inflammatory compounds of the present invention include, for example, members of the tetracycline family, opiate agonists, lipoxygenase inhibitors, cyclooxygenase inhibitors (*e.g*., cyclooxygenase-1 (COX-1) selective inhibitors, cyclooxygenase-2 (COX-2) selective inhibitors and non-selective cyclooxygenase inhibitors), interleukin receptor antagonists, NMDA receptor antagonists, inhibitors of nitric oxide or inhibitors of the synthesis of nitric oxide, non-steroidal anti-inflammatory agents, steroidal anti-inflammatory compounds or cytokine-suppressing anti-inflammatory agents

In one embodiment, the anti-inflammatory compound may be a member of the tetracycline family. The language "member of the tetracycline family" includes many compounds with a similar ring structure to tetracycline. Examples of tetracycline compounds include: oxytetracycline, demeclocycline, methacycline, minocycline, sancycline, chelocardin, rolitetracycline, lymecycline, apicycline; clomocycline, guarnecycline, meglucycline, mepylcycline, penimepicycline, pipacycline, etamocycline, penimocycline, *etc*. Other derivatives and analogues comprising a similar four ring structure are also included (See Rogalski, "Chemical Modifications of Tetracyclines," the entire contents of which are hereby incorporated herein by reference). Table 1 depicts tetracycline and several known members of the tetracycline family.

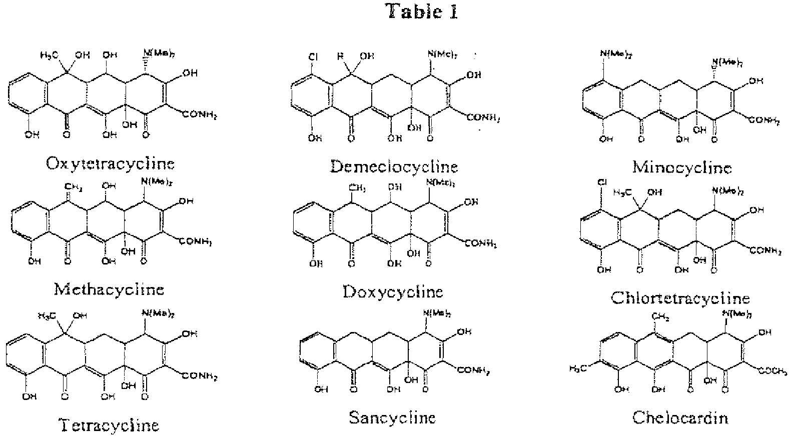

In one embodiment, the member of the tetracycline compound is selected from the group consisting of oxytetracycline, demeclocycline, minocycline, methacycline, doxycyciine, chlortetracycline, tetracycline, sancycline, chelocardin and pharmaceutically acceptable derivatives thereof. In one embodiment, the member of the tetracycline family is minocycline. The dosage of the member of the tetracycline family may be between about 50 and 500 mg per day. In one embodiment, the dosage is 400 mg. In another embodiment, the dosage is about 100 mg per day.

In another embodiment, the anti-inflammatory may be a cyclooxygenase-2 (COX-2) selective inhibitor. The language "cyclooxygenase-2 (COX-2) selective inhibitor" refers to compounds that selectively inhibit the cyclooxygenase-2 enzyme over the cyclooxygenase-1 enzyme. Suitable COX-2 inhibitors include, for example, 2-(4-ethoxy-phenyl)-3-(4-methanesulfonyl-phenyl)-pyrazolo[1,5-b]pyridazine, CDC-501, celecoxib, COX-189, CS-179, CS-502, D-1367, 4-(2-oxo-3-phenyl-2,3-dihydrooxazol-4-yl) benzenesulfonamide, darbufelone, DFP, DRF-4367, etodolac, flosulide, JTE-522 (4-(4-cyclohexyl-2-methyl-5-oxazolyl)-2-fluorobenzenesulfonamide), L-745337, L-748731, L-748780, L-768277, L-776967, L-783003, L-791456, L-804600, L₋748706, meloxicam, MK663 (etoricoxib), nimesulide, NS-398, parecoxib, 1-methylsulfonyl-4-(1,1 -dimethyl-4-(4-fluorophenyl)cyclopenta-2,4-dien-3- yl)benzene, DuP-697, L-761066, 4-(1,5-dihydro-6-fluoro-7-methoxy-3-(trifluoromethyl)-(2)-benzothiopyrano-(4,3-c)pyrazolyl-1-yl)benzenesulfonamide, 4,4-dimethyl-2-phenyl-3-(4-methylsulfonyl)phenyl)cyclobutenone, 4-amino-N-(4-(2-fluoro-5-trifluoromethyl)-thiazol-2-yl)benzene sulfonamide, meloxicam, 1-(7-tert-butyl-2,3-dihydro-3,3-dimethyl-5-benzo-furanyl)-4-cyclopropyl-butan-1-one, rofecoxib, RS-1 13472, RWJ-63556, RS-57067, S-2474, S-33516, SC-299, SC-5755, SC-57666, SC-58125, lumiracoxib valdecoxib, parecoxib sodium, APHS, UR-8877, UR-8813, UR-8880 and pharmaceutically acceptable derivatives thereof. In one embodiment, the COX-2 selective inhibitor is celecoxib. In another embodiment, the dosage of the COX-2 selective inhibitor is between about 100 and 500 mg per day. In a further embodiment, the dosage of the COX-2 selective inhibitor is about 400 mg per day.

In one embodiment, the method of treating a neurological disorder in a subject further comprises administering to the subject an effective amount of one or more therapeutic agents including, but not limited to, co-enzyme Q₁₀, ethyl-eicosapentanopic acid, a glutamate antagonist (e.g., riluzole, lamotrigine or remacemide) or phenylbutyrate.

The language "in combination with" an anti-inflammatory compound includes co-administration of creatine monohydrate and dextrose with an anti-inflammatory compound, administration of creatine monohydrate and dextrose first, followed by administration of an anti -inflammatory compound, and administration the anti-inflammatory compound first, followed by administration of creatine monohydrate and dextrose. The creatine monohydrate and dextrose can be administered substantially at the same time as the anti-inflammatory compound or at substantially different times as the anti-inflammatory compounds. In one embodiment, the creatine monohydrate, dextrose and anti-inflammatory agent are contained in one dosage form. In another embodiment, the creatine monohydrate and dextrose are in one dosage form and the anti-inflammatory agent is in a second dosage form.

Optimal administration rates for a given protocol of administration of creatine monohydrate and dextrose and/or the anti-infiammatory compound can be readily ascertained by those skilled in the art using conventional dosage determination tests conducted with regard to the specific compounds being utilized, the particular compositions formulated, the mode of application, the particular site of administration and the like. In one embodiment, the creatine monohydrate and dextrose and/or the anti-inflammatory compound are administered at least once a day, for example, twice a day or three times a day.

The term "subject" is intended to include living organisms susceptible to having neurological disorders (*e.g*., mammals). Examples of subjects include humans, dogs, cats, horses, cows, goats, rats and mice. The term "subject" also includes include transgenic species. In one embodiment, the subject is a human.

In one embodiment, the invention pertains to a pharmaceutical composition comprising a composition of creatine monohydrate and dextrose combination with an anti-inflammatory compound. In one embodiment, the pharmaceutical composition further comprises a pharmaceutically acceptable carrier. In yet another embodiment, the creatine monohydrate, dextrose and the anti-inflammatory compound may be administered together in one pharmaceutical composition or in separate pharmaceutical compositions. In a further embodiment, the pharmaceutical composition may comprise a dosage of riluzole. The dosage of riluzole may be administered together in one pharmaceutical composition with the dosage of creatine monohydrate and/or the anti-inflammatory compound or may be in separate pharmaceutical compositions.

In yet another embodiment, the invention pertains to a kit comprising a container of creatine monohydrate and dextrose and instructions for administering the creatine monohydrate and dextrose to treat a subject for a neurological disorder. In a further embodiment, the invention pertains to a kit comprising a container of creatine monohydrate, dextrose and an anti-inflammatory compound and instructions for administering the creatine monohydrate, dextrose and the anti-inflammatory compound to treat a subject for a neurological disorder.

The phrase "pharmaceutically acceptable carrier" includes a pharmaceutically acceptable material, composition or vehicle, such as a liquid or solid filler, diluent, excipient, solvent or encapsulating material, involved in carrying or transporting creatine monohydrate and/or an anti-inflammatory compound within or to the subject such that it can performs its intended function. Typically, such compounds are carried or transported from one organ, or portion of the body, to another organ, or portion of the body. Each carrier must be "acceptable" in the sense of being compatible with the other ingredients of the formulation and not injurious to the subject. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

The pharmaceutical compositions of the present invention may conveniently be presented in unit dosage form and may be prepared by any methods well known in the art of pharmacy. The amount of active ingredient which can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Those skilled in the art related to the present invention will be better able to determine an appropriate dosage and overall dosage regime when taking a number of factors into consideration. For example, the size, weight and condition of the patient must be considered as must be the responsiveness of the patient and their disorder to the particular therapy. In one embodiment, the dosage is from 0.001 µg to 100 g and may be administered once or several times daily, weekly, monthly or yearly, or even every 2 to 20 years. In one embodiment, a suitable dose of a compound of the invention will be that amount of the compound which is the lowest dose effective to produce a therapeutic effect. In another embodiment, a suitable dose of a compound of the invention will be an effective daily dose, which includes the lowest daily dose effective to produce a therapeutic effect. The effective daily dose of the active compound may be administered as two, three, four, five, six or more sub-doses administered separately at appropriate intervals throughout the day, optionally, in unit dosage forms.

The language "effective amount" of the compound is that amount necessary or sufficient to treat, prevent or ameliorate a neurological disorder in a subject. The effective amount can vary depending on such factors as the size and weight of the subject, the type of illness, *etc.* One of ordinary skill in the art would be able to study the aforementioned factors and make the determination regarding the effective amount of creatine monohydrate and/or the anti-inflammatory compound without undue experimentation.

Formulations of the invention include those suitable for oral, nasal, topical, transdermal, buccal, sublingual and/or parenteral administration. The formulations may conveniently be presented in unit dosage form and may be prepared by any methods well known in the art of pharmacy. Methods of preparing these formulations or compositions include the step of bringing into association creatine monohydrate and/or an anti-inflammatory compound, with the carrier and, optionally, one or more accessory ingredients. In general, the formulations are prepared by uniformly and intimately bringing into association a compound of the invention with liquid carriers, or finely divided solid carriers, or both, and then, if necessary, shaping the product.

Formulations of the invention suitable for oral administration may be in the form of capsules, cachets, pills, wafers, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the invention as an active ingredient. A compound of the invention may also be administered as a bolus, electuary or paste.

In solid dosage forms of the invention for oral administration (capsules, tablets, wafers, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active incredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of creatine monohydrate and/or an anti-inflammatory compound include pharmaceutically acceptable emulsions, microemulsions, solutions, suspensions, syrups and elixirs, In addition to the active ingredient, the liquid dosage forms may contain inert dilutents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for transdermal administration of compounds of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required. The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to creatine monohydrate and/or an anti-inflammatory compound, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of creatine monohydrate and/or an anti-inflammatory compound to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Pharmaceutical compositions of this invention suitable for parenteral administration comprise creatine monohydrate and/or an anti-inflammatory compound in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganism may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a compound, it is desirable to slow the absorption of the compound from subcutaneous or intramuscular injection. This may be accomplished by the use of a liquid suspension of crystalline or amorphous material having poor water solubility. The rate of absorption of the compound then depends upon its rate of dissolution which, in turn, may depend upon crystal size and crystalline form. Alternatively, delayed absorption of a parenterally-administered compound form is accomplished by dissolving or suspending the compound in an oil vehicle.

Injectable depot forms are made by forming microencapsule matrices of the compounds of the invention in biodegradable polymers such as polylactide-polyglycolide. Depending on the ratio of compound to polymer, and the nature of the particular polymer employed, the rate of compound release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the invention may be given orally, parenterally or topically. They are, of course, given by forms suitable for each administration route. For example, they are administered in tablets or capsule form, by injection, inhalation, eye lotion, ointment, suppository, and etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systematically," "peripheral administration" and "administered peripherally" as used herein mean the administration of a compound, drug or other material other than directly into the central nervous system, such that it enters the subject's system and, thus, is subject to metabolism and other like processes, for example, subcutaneous administration.

Creatine monohydrate and/or dextrose and/or an anti-inflammatory compound may be administered to humans and other animals for therapy by any suitable route of administration, including orally, nasally, as by, for example, a spray, parenterally, intracisternally and topically, as by powders, ointments or drops, including buccally and sublingually.

In one embodiment, the invention also pertains to a packaged pharmaceutical composition for the treatment of a neurological disorder (*e.g.,* Parkinson's disease, Huntington's disease and amyotrophic lateral sclerosis) the composition comprises about 5 grams of creatine monohydrate and about 2 grams of dextrose and instructions for treating the neurological disorder. In a further embodiment, the instructions include instructions for administration of the pack aged pharmaceutical composition (e.g., instructions for once a day administration, twice a day administration, etc.)

### Exemplification of the Invention

### Example 1: Effect of Creatine Monohydrate on Huntington's Disease

### Methods

*Study design.* Sixty-four subjects were enrolled at four sites. Eligible subjects were randomized to 8 g/day of creatine monohydrate or placebo by computer-generated blocked randomization with site stratification. Treatment was administered as chewable wafers twice daily for 16 weeks. A medical monitor and independent safety committee reviewed clinical data monthly. Consent, study procedures, and case report forms were approved by the institutional review board at each site. Blood samples for analysis of serum biomarkers were obtained with consent from 30 age-appropriate individuals without neurological illnesses.

*Eligibility criteria.* Eligible subjects were 18 years of age and older with a diagnosis of Huntington's disease confirmed by genetic testing, a total functional capacity score of ≥ 5, and a caregiver to witness consent and monitor compliance. Exclusion criteria included previous creatine exposure within 30 days of baseline; underlying hematological, hepatic, or renal disease; screening white blood cell count < 3,800/mm³; creatinine > 2.0 mg/dL or alanine aminotransferase greater than twice the upper normal limit; or unstable medical/psychiatric illness.

*Study protocol.* Subjects were screened within 25 days of randomization (baseline visit). Screening included assessment of eligibility criteria, medical history, physical examination. Unified Huntington's Disease Rating Scale (UHDRS), EKG, DNA analysis, complete blood count, chemistry panel, and urinalysis. Subjects were on study drug (creatine or placebo) for 16 weeks, followed by an 8-week washout. Study visits occurred at baseline, weeks 8, 16, and 24 with telephone contacts at weeks 1, 10 and 20. Physical examination was done at screening and week 16. UPDRS scores, vital signs, adverse event assessment, and safety laboratory tests were repeated at all visits. Blood was collected for serum creatine levels and serum 8-hydroxy-2'-deoxyguanosine (8OH2'dG), a measure of oxidative injury affecting DNA. The later was analyzed in the samples from two sites. Magnetic resonance spectroscopy (MRS; STEAM; TR/TE = 6000/20 milliseconds) of frontal cortex (voxel size = 56 cc) and occipital cortex (voxel size = 18 cc) performed at one site was analyzed for this report. Subject compliance was assessed by wafer counts.

*Statistical analysis.* The primary outcome measure was tolerability. Subjects who did not complete week 16 or required more than one drug suspension or any suspension exceeding 7 days were considered treatment failures. Within 27 subjects per group, there was a 94% power to detect an absolute difference of 50% or more between the placebo group and the active treatment group assuming a 15% dropout rate. In accordance with intent to treat, all randomized subjects were included in safety and tolerability analysis. Tolerability was assessed by comparing the proportion of treatment failures in creatine and placebo groups using χ² test with continuity correction. As the primary interest was in detecting intolerability in the active group, a one-sided test was used (significance = 0.05). This was also applied to assess adverse events and laboratory abnormalities. The laboratory score changes from screening were analyzed by repeated-measures analysis of variance (ANOVA), and the differences between visits were analyzed by paired *t* test. Demographic and baseline variables were summarized for each group, and comparisons made using Fischer's exact test and continuous variables were compared using t test. Changes in UHDRS motor, cognitive, behavioral, and functional component subscores were measured at baseline and weeks 8, 16, and 24 and analyzed by mixed-model ANOVA.

### Results

*Enrollment and baseline characteristics.* Sixty-four of 69 subjects screened were enrolled, 32 per treatment group. There were no differences between the group (Table 2), with the exception of a higher rate of depression in the placebo group (p < 0.02). Normal control donating blood for 8OH2'dG determinations consisted of 19 men and 11 women with an average age of 43.6 and a range of 21 to 78, indistinguishable from the subjects with Huntington's disease.

**Table 2**

| **Variable** | **Creatine (n = 32)** | **Placebo (n = 32)** |
|---|---|---|
| Gender (F:M) | 12:20 | 14:18 |
| Age, y (range) | 44.7 (19-62) | 47.9 (27-73) |
| Duration of illness, per rater, y | 7.9 (3.5) | 6.1 (5.4) |
| CAG repeat number | 44.8 (2.6) | 45 (5.5) |
| Weight, kg | 76.5 (15.3) | 77.0 (15.9) |
| Total functional capacity | 9.0 (2.3) | 9.3 (1.7) |
| Independence score | 81.4 (11.4) | 83.1 (9.2) |
| Total chorea score | 13.3 (5.7) | 13.4 (5.2) |
| Total motor score | 46.8 (16.9) | 42.8 (16.5) |
| Symbol Digit Modalities Test (*p* < 0.05) | 21.0 (8.5) | 28.3 (10.9) |
| Verbal Fluency Test (*p* < 0.05) | 17.56 (9.1) | 22 47 (12.6) |
| Stroop Interference | 23.8 (1.4) | 27.1 (11.7) |

| | | |
|---|---|---|
| Values arc means (= SD) or ratio Scale ranges (normal to most severe) include total functional capacity (13 to 0), independence score (100 to 10), total chorea scores (0 to 28), total dystoma score (0 to 20), total motor score (0 to 124) For cognitive tests, higher scores are better: Symbol Digit (number of correct responses in 90 seconds), Verbal Fluency (number of correct responses in 3 minutes), Stroop interference (number of correct responses in 45 seconds). CAG = cytosine-adenine-guanine trinucleotide repeat numbers. | | |

*Tolerability of creatine.* Creatine at a dose of 8g/day was well tolerated. Treatment assignment did not affect the likelihood of study completion (one-sided Fisher's test, p = 0.5). Of five subjects who terminated the study, two were on placebo (fall, difficulty with travel) and three on creatine (no specified reason, dysgeusia, dysphagia). There were six serious adverse events in three subjects in the placebo group (fall, vomiting, incorrect study drug) and three in the creatine group (fall, depression requiring hospitalization, suicidal ideation). The incidence of adverse events was similar between groups (Table 3), and differences in the occurrence of any particular adverse event were insignificant. Compliance was comparable between treatment groups (90% vs. 89%). Body mass index and UHDRS subscores were compared with baseline using paired t test and did not change with treatment. There were no significant laboratory abnormalities with the exception of mild reversible serum creatinine and alkaline phosphatase elevations. Serum creatinine (0.88 ± 0.19 to 1.04 ± 0.24, p < 0.01) increased by week 8 in the creatine group, remained elevated at week 16, and returned to baseline at washout. These elevations were statistically but not clinically significant and are consistent with creatinine being the primary metabolite of creatine.

**Table 3**

| **Variable** | **Creatine (n = 32)** | **Placebo (n = 32)** |
|---|---|---|
| Study withdrawal before week 24 | 2 | 3 |
| All adverse events: mild, moderate and severe, no. (%) | 26 (86.9) | 29 (90.7) |
| Serious adverse events | 3 | 3 |
| Depression | 4 | 5 |
| Fall | 3 | 3 |
| Irritability | 3 | 0 |
| Lethargy | 2 | 0 |
| Dizziness | 1 | 1 |
| Increased movements | 1 | 1 |
| Vomiting | 1 | 2 |
| Dysphagia | 1 | 0 |
| Apathy | 1 | 1 |
| Cough | 1 | 1 |
| Hematuna | 1 | 1 |

| | | |
|---|---|---|
| Values are frequencies and percentages. Differences between groups are not significant. | | |

*Effect of creatine or serum and brain biomarkers.* Average serum creatine levels in the treatment group rose from 46.4 µmol/L (SD = 35.7) at baseline to 382.5 µmol/L (SD = 259.9) at week 16 and back to 5 1.8 µmol/L (SD = 29.5) at washout; creatine concentrations were unchanged in the placebo group at each time point. By MRS, absolute creatine concentrations, estimated from the pool of total metabolites, increased by 13% (p < 0.05) in the occipital cortex and by 7.5% in the frontal cortex (p < 0.05) by week 16 in subjects on creatine and returned to baseline at week 24. Brain creatine was unchanged in the placebo group.

Figure 1 is a scatterplot that illustrates individual changes in 8OH2'dG (pg/mL) levels at week 16 in the 32 subjects placebo and 32 creatine treated subjects. Serum 8OH2'dG levels were significantly (p *<* 0.0001) elevated at baseline in subjects with Huntington's disease (45.29 pg/mL, SD = 14.62) compared to normal controls (13.5 pg/mL, SD = 4.04). Treated subjects had an average reduction of 8OH2'dG of 9.11 pg/mL (SD = 13.93), whereas placebo subjects averaged an increase of 5.87 pg/mL (SD = 11.52). The reduction from baseline to 16 weeks was significant in the treated group compared to the placebo group. (p < 0.0042).

### Summary

Creatine, at a dose of 8g/day, was well tolerated by subjects with Huntington's disease. Clinical (UHDRS) measures were unchanged over the treatment course. Adverse events occurred with equal frequency in creatine and placebo groups. Reversible but mild increases in serum creatinine and alkaline phosphatase were seen by week 8 in subjects on creatine, returning to baseline levels at the washout visit. Serum and brain creatine concentrations rose significantly during treatment and returned to baseline levels after washout, indicating systemic and brain bioavailability of ingested creatine. The improvement of elevated 8OH2'dG observed suggests reduced oxidative injury in subjects with Huntington's disease on creatine treatment. This finding is consistent with oxidative injury to DNA being an indicator of disease activity and contributing to pathogenesis.

Overall, this example demonstrated that 8 grams daily of creatine is safe and tolerable, elevates blood and brain levels of creatine and positively impacts biomarkers of oxidative injury and neurodegeneration.

### Example 2: Randomized, double-blind, clinical trial on creatine and minocycline in early Parkinson's disease (PD)

### Methods

*Study Design and Randomization.* This example was a single arm futility study designed to assess creatine and minocycline. A placebo arm for calibration was included in the example. Eligible subjects were randomly assigned in a 1:1:1 fashion to receive 1) 10g/day of creatine monohydrate and placebo minocycline, 2) placebo creatine monohydrate and 200mg/day of minocycline, or 3) placebo creatine monohydrate and placebo minocycline. The primary futility analysis was at 12 months of follow-up, but each subject was followed for 18 months for additional safety information. Subjects and investigators were kept blinded to treatment group.

*Participants.* Subjects were men and women age 30 and over who had a diagnosis of PD but did not require medications for the management of their symptoms. Two of the three cardinal manifestations of PD (tremor, rigidity, and bradykinesia) were required; these findings had to be asymmetric. The diagnosis of PD must have been made within 5 years of randomization. Women of childbearirag potential were required to use adequate birth control and have a negative pregnancy test at baseline. Subjects were excluded if they had any secondary causes of parkinsonism, such as drug induced parkinsonism or structural lesions; had atypical parkinsonian syndromes; gait freezing or impairment in postural reflexes; had prior stereotaxis surgery for PD; used creatine, minocycline, or any investigational agent within 90 days prior to randomization; had known hypersensitivity to creatine or minocycline; used CoQ₁₀ in doses greater than 300 mg 90 days prior to randomization; or had any clinically significant medical condition that could interfere with the subject's ability to safely participate in the study or be followed.

*Dosages.* Creatine was administered as 5 g sachets mixed with 8 ounces of liquid taken twice a day and minocycline was administered as 100 mg capsules taken twice a day. Both were taken with meals.

*Outcome measures.* The primary, prespecified outcome measure was the change in the total Unified PD Rating Scale (UPDRS) score from baseline to either the time at which there was sufficient disability to warrant symptomatic therapy for PD or 12 months, whichever came first. Disability was assessed by the sight investigator, based on impairment in ambulation, activities in daily living, and occupational status. The mean change in total UPDRS for each treatment group was compared to a prespecified futility threshold of a 30% reduction in the historically derived change in the total UPDRS, which was based on a placebo arm of a previous clinical trial. Tolerability was defined as the proportion of subjects taking study drug for the full 12 months. All severe adverse events (SAEs) were reviewed by the study medical monitor and an independent medical monitor. Both the site investigator and medical monitors assessed the potential relationship between SAEs and study drug.

*Study Procedures.* At the screening visit, the purpose and potential risks were explained to potential subjects and each subject gave written consent. Subjects then had a baseline medical history, physical examination, and underwent the UPDRS. Blood was obtained for serum chemistry and complete blood count. Participants were reevaluated at 1, 3, 6, 9, and 12 months (±6 days) after the baseline visit using the battery of clinical scales and blood was drawn again at 6 and 12 months.

*Sample size and statistical analysis* The sample size estimation was based on data from patients on placebo/tocopherol participating in the Deprenyl and Tocopherol Antioxidant Therapy of Parkinsonism trial (DATATOP), a large cohort of newly diagnosed patients with PD similar to the planned study population. The DATATOP study met the Pocock criteria for the use of historical controls. The observed mean change from baseline of total UPDRS in placebo/tocopherol patients (13 months ± 30 days) was 10.65 (SD 10.4), The threshold value was defined as 30% less progression on the total UPDRS than the 10.65 unit change in DATATOP, or 7.46. A sample size of 58 per group provides power greater than 85% to reject the null hypothesis of non-futility if in fact the true mean total UPDRS worsening is greater than the threshold of 7.46 at the design alternative of 10.65. As with most clinical studies, a certain degree of noncompliance (including subject withdrawl or lost-to-follow-up) was expected. Assuming the non-compliance rate to be minimal at 5%, the required sample size was increased to 65 per treatment arm to account for the noncompliance in the intent-to-treat analysis. For each study arm, the set of statistical hypotheses was as follows.H₀: Δᵢ ≤ 7.46 vs Hₐ, Δᵢ >7.46(=10.65), where Δᵢ is the mean change score (total UPDRS at 12 months or at the time of initiation of symptomatic therapy-total UPDRS at baseline) for the treatment arm 1 and 7.46 is the maximum mean increase (worsening) in the score between baseline and 12 months sufficient to warrant further evaluation of the drug in the Phase II trial. The hypothesis was tested with a one-sample t test at one-sided alpha level of 0.10. If the null hypothesis was rejected (p ≤ 0.1) then the drug would be considered futile for further testing in a Phase III trial.

A secondary analysis of the primary outcome was planned, if the mean change in the total UPDRS score observed in the calibration placebo group falls outside of the 95%CI of the historical control-group mean change score of 10.65 (±1.02). The historical rate derived from DATATOP would be updated by incorporating the information from the calibration placebo group using Bayesian methods to derive a posterior mean. The futility threshold would be recomputed as 70% of this posterior mean and a one-sample t test would be performed for each active treatment arm

Analysis of the primary outcome was conducted under the intent-to-treat principle where all randomized subjects are included in the analyses. For the small proportion of subjects who were lost to followup, the UPDRS change scores were imputed using the worst change score observed within their respective treatment groups. Exploratory analyses included multiple imputation to account for missing values and a sensitivity analysis which used a 30% reduction from the observed calibration placebo value to recomputed the futility threshold value. No two-sample comparisons were made between placebo and treatment groups as the study was not designed or powered for this type of analysis. A two sample comparison would be underpowered compared to the planned futility analysis.

### Results

*Subjects enrolled.* Eligible subjects (200) were randomized to one of three treatment groups: Group I received creatine; Group 2 received minocycline; Group 3 received placebo. The treatment groups were similar at baselines on demographic variables and total UPDRS and UPDRS subscores.

*Futility.* Compliance with study visits was high and only two patients in the creatine group and no patients in the minocycline group had missing values requiring imputation. The mean change (SD) in total UPDRS from either baseline to 12 months or the time at which symptomatic therapy was needed was 5.6 (8.69) for the creatine group and 7.09 (8.71) for the minocycline group (Table 4). The observed progression in both the creatine and minocycline groups did not exceed the predetermined futility threshold. Therefore, the null hypothesis that the means were less than or equal to the threshold value of 7.46 (30% less than the 10.65 DATATOP historical rate of progression) could not be rejected for creatine (p = 0.96) or minocycline (p = 0.63). Creatine and minocycline could not be rejected as futile using this analysis and therefore met the criteria for consideration of further clinical testing. Using multiple imputation instead of worst change score for the group to account for missing observations yielded similar results.

**Table 4**

| **Treatment group** | **Mean (SD)** | **95% CI** |
|---|---|---|
| Primary Analysis* | | |
| Total UPDRS | | |
| Creatine | 5.6 (8.69) | (3.48, 7.72) |
| Minocycline | 7.09 (8.71) | (4.95, 9.23) |
| Placebo (Calibration) | 8.39 (9.76) | (6.01, 10.8) |
| DATATOP Placebo/Tocopherol | 10.65 (10.4) | (9.63, 11.67) |

| | | |
|---|---|---|
| Worst change score for the group is used to impute missing values *Primary analysis compares each treatment group to the futility threshold, or 70% historical control, which equals 7.46 | | |

The calibration placebo group mean change of 8.39 (9.76) fell outside the 95% CI for the DATATOP historical control of 9.63 to 11.67. The historical control was updated using the observed placebo information and the threshold value decreased to 7.2. Against this comparison, neither creatine (p = 0.93) nor minocycline (p = 0.54) could be rejected as futile. Additionally, an exploratory sensitivity analysis was performed based on the calibration placebo group. Using only data from the placebo group, the threshold value was recomputed as 30% less than 8.93 and the futility threshold decreased to 5.87. With a threshold of 5.87, neither creatine (p = 0.6) not minocycline (p = 0.13) could be rejected as futile, although minocycline was close to the prespecified alpha level of 0.10.

Combining all three arms of the study, 96 (48%) subjects required symptomatic therapy during the course of the trial. Subjects requiring symptomatic therapy had greater changes in their UPDRS scores than those who completed 12 months without the addition of symptomatic therapy. The 104 (52%) subjects who did not receive symptomatic therapy (or terminated prior to completion) had a mean change of 4.35 (8.83) on their total UPDRS score over 12 months compared to 12.36 (8.86) for the 22 subjects starting levodopa and 8.98 (8.84) for the 47 subjects starting dopamine agonists.

*Safety and tolerability.* Creatine, minocycline and the matching placebo formulations were generally well tolerated, although minocycline was less well tolerated than creatine. Study drug was prematurely discontinued in 5 cases (7%) in the creatine arm, 14 (21%) in the minocycline arm and 4 (6%) in the calibration placebo arm. There were 8 patients with dose reductions and 8 patients with temporary suspensions in the creatine group, 15 patients with dose reductions, and 15 patients with temporary suspensions in the minocycline group, and 11 patients with does reductions and 8 patients with temporary suspensions in the placebo group, some of whom eventually discontinued study treatment.

The three most commonly reported adverse effects across the three treatment groups were upper respiratory symptoms (26%), joint pain (19%), and nausea (17%). There were six incidents of elevated serum creatine in the creatine group; three of these occurring in two subjects, were judged to be clinically significant.

Fourteen SAEs occurred during the study. Five occurred in the creatine group (cardiomyopathy, coronary artery disease, fatal motorcycle accident, myocardial infarction, and spinal stenosis surgery). Six occurred in the minocycline group (two cases of coronary artery disease, two cases of pneumonia, syncopem and squamous cell carcinoma). Three occurred in the calibration placebo group (colon cancer, pneumonitis, renal calculus). Of the events in the two active treatment groups, only the squamous cell carcinoma in the minocycline group was coded by blinded investigators as possibly related to the study interventions. None of the SAEs were judged as definitely or probably related by the independent medical monitor.

### Summary

This randomized, double blind, futility trial showed that neither creatine nor minocycline were futile in a 12 month evaluation of the clinical progression of PD. The effects of creatine on UPDRS progression were robust to a range of threshold values, whether based on historical control data updated historical control, or calibration placebo group. The mean progression in the minocycline group was above the futility threshold based on the calibration placebo group (5.87) in the sensitivity analysis.

The effects of creatine on UPDRS progression were robust to the range of threshold values tested. Overall, the study showed that creatine slows down the progression of PD and can be used as a therapy for subjects with PD.

### Example 3_{:} High-dose creatine in symptomatic Huntington's disease.

A two-phase open-label study was conducted to better determine an optimal dose of creatine symptomatic subjects with Huntington's disease. A dose-escalation study (10-40 grams per day) was conducted to determine the maximally tolerated dose (MTD) followed by a de-escalation phase to assess whether brain and serum levels of creatine might be maximal at doses lower than the MTD. Ten subjects were enrolled and followed prospectively for two weeks at each done level increasing in 5-gram increments during dose escalation that lasted 13 weeks. Assessments at each visit included UHDRS, EKG, vital signs, clinical safety and research labs. MRI spectroscopy was conducted prior to baseline at peak done (40 grams) and one month after de-escalation to either 30 or 15 grams daily. To determine an optimal dose, pharmacokinetic as well as clinical data were considered. Once the maximal dose was reached, subjects were as signed one of two lower doses previously taking (15 grams a day (n = 5) or 30 grams a day (n = 5)). Serum creatine was assessed at baseline, at the end of each 2-week dose escalation step and at the end of the de-escalation phase to assess the correspondence between serum and brain levels of creatine at steady state.

### Results

All subjects tolerated up to 40 grams/day of creatine during the does escalation and there were no withdrawals. However, there were increased numbers of adverse events at 35 and 40 grams daily, chiefly low-grade nausea and diarrhea. There were no serious adverse events, no significant laboratory abnormalities and no EKG alterations at any of the doses. Although individual subject's creatinine and BUN levels remained within normal clinical ranges, there were changes in average levels, Average creatinine levels increased in a dose-dependent manner from baseline (1.0 ± 0.2 mg/dl) to 30 grams a day (1.28 ± 0.16 mg/dl). Creatinine levels remained stable up to 40 grams/day but decreased in the de-escalation phase. BUN levels did not increase with higher doses during dose escalation and actually decreased somewhat at 15, 25, 30 and 40 grams daily creatine doses perhaps because of an emphasis placed on good hydration. Serum creatine levels increased with each dosage step until they reached a plateau at 30-35 grams/day and actually declined at 40 grams daily. Changes in UHDRS subscores were not significant. Some subjects felt worse, however, on 35 and 40 grams daily, which could have been due to GI side effects. Brain levels of creatine in the frontal cortex, as assessed by MRI spectroscopy indicated continued increases in brain creatine throughout the dosage range indicating that these high doses are not saturating brain levels. There was a dose dependent suppression of 8OH2'dG to levels greater than seen when subjects were administered 8 grams of creatine per day (see Example 1.). Higher doses were associated with further suppression and doses greater than about 25 grams daily were associated with maximum suppression of serum 8OH2'dG levels similar to normal controls. These data suggested that doses higher than 10 grams daily are safe, tolerable and have greater peripheral and brain bioavailability, and are associated with further suppression of a disease related mechanism (oxidative stress as measured by 8OH2'dG), Based on all of this data, 30 grams daily provided excellent tolerability, high bioavailability and maximal biomarker suppression.

Subjects at the end of this study were given the option to continue a long-term study to evaluate the long-term safety and tolerability of high dosage of creatine. Subjects have been followed for nine months on creatine. There have been no significant clinical changes (UPDRS subscores) up to 9 months (see Table 5) and there have been no serious adverse effects. Adverse events have been infrequent and mild to moderate in grade and 30 grams daily creatine has been well-tolerated for 9 months. Laboratory abnormalities have been mild. One subject had a Grade 2 (moderate) SGPT level after one-month on 30 grams/day, but his normalized by month 3 without dose adjustment. Thus long term administration of 30 grams daily of creatine appears to be safe and well-tolerated.

*High dose creatine slows cortical thinning.* Morphometric neuroimaging was performed in all subjects. Longitudinal data from up to three years prior to initiating creatine was available on 6 or the 10 subjects. The rate of thinning of cortical regions in HD was modeled based on the longitudinal data and a change in rate was determined for each region of the group while on creatine. Creatine reduced the rate of thinning in almost every region, and the rate of change was determined for each region for the group while on creatine. Creatine reduced the rate of thinning in almost every region, and the rate of change was statistically significant for several regions and amounted to about a 30% slowing. There was no effect on the rate of caudate volume loss (p = 0.67), rate of putamen volume loss, or on total brain volume. The lack of an effect on striatal volumes likely reflects the fact that in these individuals, caudate and putamen volumes were already reduced by more than half and the lack of change in whole brain by the fact that small proportion of the brain is represented by the detected change.

**Table 5**

| Visit | Baseline (Week 0) | De-escalation Baseline | Week 24 (Visit 4) |
|---|---|---|---|
| N (dose) | 10 | 5 (15 g/day) | 10 30 g/day |
| | | 5 (30 g/day) | |
| Weight (kg) | 75.9(14.7) | 83.3 (16.4) | 78.1 (17.5) |
| | | 75.1 (8.6) | |
| BMI (kg/m²) | 26.7 (3.2) | 27.2 (1.8) | 26.8 (17.5) |
| | | 27.2 (3.6) | |
| Total Functional | 8.9 (2.1) | 8.8 (1.8) | 8.7 (2.2) |
| Capacity | | 9.8 (3.4) | |
| Independence Score | 85.0 (11.8) | 75.0 (8.7) | 82.5 (8.9) |
| | | 82.5 (14.4) | |
| Total Chorea Score | 6.0 (2.9) | 5.5 (1.7) | 7.0 (4.1) |
| | | 7.8 (4.3) | |
| Total Functional | 19.6 (4.0) | 10.6 (9.8) | 19.6 (4.1) |
| Assessment | | 15.8 (10.2) | |
| Symbol Digit | 31.4 (13.1) | 29.0 (10.6) | 30.56 (12.63) |
| Modalities Test | | 34.0 (20.5) | |
| Verbal Fluency Test | 24.5 (14.4) | 15.8 (16.0) | 26.6 (17.67) |
| | | 28.3 (15.8) | |
| Stroop Interference | 32.2 (14.4) | 23.8 (13.1) | 31.3 (15.23) |
| | | 40.0(19.6) | |
| Stroop Word Reading | 67.0 (28-4) | 56.0 (24.3) | 62.7 (24.11) |
| | | 90.0 (24.2) | |
| Stroop Color Naming | 55.9 (24.9) | 52.8 (22.9) | 5 1.7 (21.37) |
| | | 57.8 (24.2) | |

### Example 4. Combined Survival Analysis in Amyotrophic Lateral Sclerosis Trials

Analysis of two placebo-controlled randomized trials of 5gm creatine per day in ALS suggests that creatine may have a beneficial effect on survival The two trials analyzed included a total of 211 patients, 103 creatine-treated and 108 placebo-treated subjects.

For this analysis, a measure of treatment effect in an estimated pooled hazard ratio was calculated. This measure gives the risk of death for treated subjects divided by the risk of death for controls. Survival analysis by treatment was also performed for each trial separately. A Cox model was used to assess the hazard ratios and survival by treatment in the pooled data, controlling for trial effect and baseline variables. Baseline characteristics of the study populations were also compared. Continuous variables were compared by treatment and trial, using the analysis of variance by putting both treatment and trial as the dependent variables. A Cochran-Mantel-Haenszel test statistic was used to compare discrete variables by treatment and trial, controlling for trial and treatment effects, respectively.

Creatine 5 gm per day improved survival in the pooled data, but the treatment effect fell just short of significance (p=0.059). The adjusted hazard ratio was 0.34 (9.5% confidence interval 0.11 to 1.04) for the creatine group relative to the placebo group. Median survival showed a two-to-three-fold improvement for patients taking creatine over placebo (87 months compared to 27 months). The effect was homogeneous between trials (p=0.062, hazard ratio 0.34, 95% confidence interval 0.11 to 1.06), suggesting no major differences in survival due to patient variables. This analysis suggests that creatine 5 gm daily prolongs median survival time in patients with ALS.

### Equivalents

Those skilled in the art will recognize, or be able to ascertain using no more than routine experimentation, many equivalents to the specific embodiments of the invention described herein. Such equivalents are intended to be encompassed by the following claims.

The entire contents of all references, patents, and patent applications cited herein are expressly incorporated by reference.

The present application also relates to the following special embodiments:
1. A method treating a neurological disorder in a human comprising administering to the human a composition comprising creatine monohydrate and dextrose, such that the neurological disorder in said human is treated.
2. The method item 1, wherein the neurological disease is Huntington's disease, Parkinson's disease or amyotrophic lateral sclerosis.
3. The method of item 1, wherein the composition comprises between about 1 and about 50 grams of creatine monohydrate.
4. The method of item 3, wherein composition comprises between about 10 and about 20 grams of creatine monohydrate.
5. The method of item 4, wherein the composition comprises about 15 grams of creatine monohydrate.
6. The method of item 3, wherein the composition comprises between about 1 and about 10 grams of creatine monohydrate.
7. The method of item 6, wherein the composition comprises about 5 grams of creatine monohydrate.
8. The method of item 1, wherein the composition comprises between about 1 and about 20 grams of dextrose.
9. The method of item 8, wherein the composition comprises between about 1 and about 10 grams of dextrose.
10. The method of item 9, wherein the composition comprises about 2 grams of dextrose.
11. The method of item 1, the composition is administered to the human at least once a day.
12. The method of item 11, wherein the composition is administered to the human at least twice a day.
13. The method of any one of items 1-12, wherein said composition comprises pharmaceutical grade creatine monohydrate.
14. A method of treating Parkinson's disease in a human comprising administering to the human a composition comprising creatine monohydrate and dextrose, such that the Parkinson's disease in said human is treated.
15. The method of item 14, wherein the composition comprises about 5 grams of creatine monohydrate.
16. The method of item 14, wherein the composition comprises about 2 grams of dextrose.
17. The method of item 14, wherein the composition is administered to the human at least twice a day.
18. The method of any one of items 14-17, wherein said composition comprises pharmaceutical grade creatine monohydrate.
19. A method of treating amyotrophic lateral sclerosis in a human comprising administering to the human a composition comprising creatine monohydrate and dextrose, such that the amyotrophic lateral sclerosis in said human is treated.
20. The method of item 19, wherein the composition comprises about 5 grams of creatine monohydrate.
21. The method of item 19, wherein the composition comprises about 2 grams of dextrose.
22. The method of any one of items 19-21, wherein the composition is administered to the human at least once a day.
23. The method of any one of items 19-22, wherein said composition comprises pharmaceutical grade creatine monohydrate.
24. A method of treating Huntington's disease in a human comprising administering to the human a composition comprising creatine monohydrate and dextrose, such that the Huntington's disease in said human is treated.
25. The method of item 24, wherein the composition comprises about 15 grams of creatine monohydrate.
26. The method of item 24 or 25, wherein the composition comprises about 2 grams of dextrose.
27. The method of any one of items 24-26, wherein the composition is administered to the human at least twice a day.
28. The method of any one of items 24-27, wherein said composition comprises pharmaceutical grade creatine monohydrate.
29. A method for treating amyotrophic lateral sclerosis in a human comprising administering a composition comprising about 5 grams of creatine monohydrate and about 2 grams of dextrose once a day to said subject, such that the amyotrophic lateral sclerosis in said subject is treated.
30. A method for treating Huntington's disease in a human comprising administering a composition comprising about 15 grams of creatine monohydrate and about 2 grams of dextrose twice a day to said subject, such that the Huntington's disease in said subject is treated.
31. A method for treating Parkinson's disease in a human comprising administering a composition comprising about 5 grams of creatine monohydrate and about 2 grams of dextrose twice a day to said subject, such that the Parkinson's disease in said subject is treated.
32. The method of any one of items 29-31, wherein said creatine monohydrate is pharmaceutical grade creatine monohydrate.
33. A method of treating a neurological disorder in a human comprising administering to said human a composition comprising creatine monohydrate and dextrose in combination with an anti-inflammatory compound, such that the neurological disorder in said human is treated.
34. The method of item 33, wherein said the composition further comprises riluzole.
35. The method of item 33 or 34, wherein the neurological disorder is Huntington's disease, Parkinson's disease, Charcot Marie Tooth syndrome, muscular dystrophy, Alzheimer's disease or amyotrophic lateral sclerosis.
36. The method of any one of items 33-35, wherein the anti-inflammatory compound is a member of the tetracycline family.
37. The method of item 36, wherein the member of the tetracycline family is selected from the group consisting of oxytetracycline, demeclocycline, minocycline, methacycline, doxycycline, chlortetracycline, tetracycline, sancycline and chelocardin.
38. The method of item 37, wherein the member of the tetracycline family is minocycline.
39. The method of item 36, wherein the member of the tetracycline family is administered in an amount of between about 50 and 500 mg.
40. The method of item 39, wherein the amount is about 400 mg.
41. The method of item 39, wherein the amount is about 100 mg.
42. The method of any one of items 33-35, wherein the anti-inflammatory compound is a cyclooxygenase-2 (COX-2) selective inhibitor.
43. The method of item 42, wherein the COX-2 selective inhibitor is selected from the group consisting of 2-(4-ethoxy-phenyl)-3-(4-methanesulfonyl-phenyl)-pyrazolo[1,5-b]pyrid- azine, CDC-501, celecoxib, COX-189, CS- 179, CS-502, D-1367, 4-(2-oxo-3-phenyl-2,3-dihydrooxazol-4-yl) benzenesulfonamide, darbufelone, DFP, DRF-4367, etodolac, flosulide, JTE-522 (4-(4-cyclohexyl-2-methyl-5-oxazolyl)-2-fluorobenzenesulfonamide), L-745337, L-748731, L-748780, L-768277, L-776967, L-783003, L-791456, L-804600, L-748706, meloxicam, MK663 (etoricoxib), nimesulide, NS-398, parecoxib, 1-methylsulfonyl-4-(1,1-dimethyl-4-(4-fluorophenyl)cyclopenta-2,4-dien-3- yl)benzene, DuP-697, L-761066, 4-(1,5-dihydro-6-fluoro-7-methoxy-3- (trifluoromethyl)-(2)-benzothiopyrano-(4,3-c)pyrazol-1-yl)benzenesulfonamide, 4,4-dimethyl-2-phenyl-3-(4-methylsulfonyl)phenyl)cyclobutenone, 4-amino-N-(4-(2-fluoro-5-trifluoromethyl)-thiazol-2-yl)benzene sulfonamide, meloxicam, 1-(7-tert-butyl-2,3-dihydro-3,3-dimethyl-5-benzowfuranyl)-4-cyclopropyl-butan-1-one, rofecoxib, RS-113472, RWJ-63556, RS-57067, S-2474, S-33516, SC-299, SC-5755, SC-57666, SC-58125, lumiracoxib valdecoxib, parecoxib sodium, APHS, UR-8877, UR-8813, UR- 8880 or a pharmaceutically acceptable derivatives thereof.
44. The method of item 43, wherein the COX-2 selective inhibitor is celecoxib.
45. The method of any one of items 42-44, wherein the COX-2 selective inhibitor is administered in an amount of between about 100 and 500 mg.
46. The method of item 45, wherein the amount is about 400 mg.
47. The method of any one item 33-46, wherein the composition comprises between about 1 and about 50 grams of creatine monohydrate.
48. The method of item 47, wherein the composition comprises about 15 grams of creatine monohydrate.
49. The method of item 47, wherein the composition comprises about 5 grams of creatine monohydrate.
50. The method of any one of items 33-46, wherein the composition comprises between about 1 and about 20 grams dextrose.
51. The method of item 50, wherein the composition comprises about 2 grams of dextrose.
52. The method of any one of items 33-51, wherein said creatine monohydrate is pharmaceutical grade creatine monohydrate.
53. A pharmaceutical composition comprising creatine monohydrate, dextrose and an anti-inflammatory compound.
54. The composition of item 53, wherein the composition further comprises riluzole.
55. The composition of item 53, wherein the composition comprises between about 1 and about 50 grams of creatine monohydrate.
56. The composition of item 55, wherein composition comprises between about 10 and about 20 grams of creatine monohydrate.
57. The composition of item 56, wherein the composition comprises about 15 grams of creatine monohydrate.
58. The composition of item 55, wherein the composition comprises between about 1 and about 10 grams of creatine monohydrate.
59. The composition of item 58, wherein the composition comprises about 5 grams of creatine monohydrate.
60. The composition of any one of items 53-59, wherein the composition comprises between about 1 and about 20 grams of dextrose.
61. The composition of item 60, wherein the composition comprises between about 1 and about 10 grams of dextrose.
62. The composition of item 60, wherein the composition comprises about 2 grams of dextrose.
63. The composition of any one of items 53-62, wherein the creatine monohydrate and dextrose are formulated in a powder.
64. The composition of item 63, wherein the powder has a particle size of between about 1000 and about 1500 microns.
65. The composition of item 64, wherein the powder has a particle size of between about 1100 and about 1200 microns.
66. The composition of item 65, wherein the powder has a particle size of about 1 190 microns.
67. The composition of any one of items 53-66, wherein said creatine monohydrate is pharmaceutical grade creatine monohydrate.
68. The composition of any one of items 53-67, wherein the composition further comprises a pharmaceutically acceptable carrier.
69. A kit comprising a container of creatine monohydrate, dextrose and an anti-inflammatory compound and instructions for administering the creatine monohydrate, dextrose and an anti-inflammatory compound to a human such that the human is treated for a neurological disorder.
70. A packaged pharmaceutical composition for treatment of Parkinson's disease, wherein the composition comprises about 5 grams of creatine monohydrate and about 2 grams of dextrose and instructions for treating Parkinson's disease.
71. The packaged composition of item 70, wherein the instructions include instructions for twice a day administration.
72. A packaged pharmaceutical composition for treatment of amyotrophic lateral sclerosis, wherein the composition comprises about 5 grams of creatine monohydrate and about 2 grams of dextrose and instructions for treating Parkinson's disease.
73. The packaged composition of item 72, wherein the instructions include instructions for once a day administration.
74. A packaged pharmaceutical composition for treatment of Huntington's disease, wherein the composition comprises about 15 grams of creatine monohydrate and about 2 grams of dextrose and instructions for treating Huntington's disease.
75. The packaged composition of item 74, wherein the instructions include instructions for twice a day administration.
76. The method of any one of items 1, 14, 19, 24, 29-31 and 33, wherein said composition slows cortical thinning of the brain.
77. The method of any one of items 1, 14, 19, 24, 29-31 and 33, wherein said composition modulates the levels of one or more biomarkers in said subject.
78. The method of item 77, wherein said modulation is a reduction in the levels of one or more biomarkers.
79. The method of item 77, wherein said biomarker is 8OH2'dG.
80. The method of item 79, wherein the level of 8OH2'dG is reduced to a level comparable to the level of 8OH2'dG in a healthy subject
81. The method of any one of items 1, 14, 19, 24, 29-31 and 33, wherein said composition modulates the levels of one or more genetic markers in said subject.
82. The method of item 81, wherein said genetic marker is pleopmorphic adenoma gene-like 1 (PLAG1) or H2A histone family, member Y (H2AFY).
83. The method of any one of items 1, 14, 19, 24, 29-31 and 33, wherein said composition further comprises co-enzyme Q₁₀, ethyl-eicosapentanopic acid, a glutamate antagonist or phenylbutyrate.
84. The method of item 83, wherein said glutamate antagonist is riluzole, lamotrigine or remacemide.

## Claims

1. A composition comprising an effective amount of creatine monohydrate for use in the treatment of amyotrophic lateral sclerosis, Parkinson's Disease or Huntington's disease.

2. The composition of claim 1, wherein the composition comprises pharmaceutically acceptable grade of creatine monohydrate.

3. The composition of claim 2, wherein the creatine monohydrate is substantially free of toxins.

4. The composition of claim 3, wherein the toxins are heavy metals, creatinine or dihydroazines.

5. The composition of any one of claims 1-4, wherein the composition comprises between about 1 and about 50 grams of pharmaceutically acceptable grade of creatine monohydrate, wherein the creatine monohydrate is substantially free of toxins.

6. The composition of claim 5, wherein the composition comprises between about 1 and about 10 grams of creatine monohydrate.

7. The composition of claim 6, wherein the composition comprises about 5 grams of creatine monohydrate.

8. The composition of any one of claims 1-7, wherein the composition comprises between about 1 and about 20 grams of dextrose.

9. The composition of claim 8, wherein the composition comprises between about 1 and about 10 grams of dextrose.

10. The composition of claim 9, wherein the composition comprises about 2 grams of dextrose.

11. The composition of any one of claims 8-10, wherein the creatine monohydrate and dextrose are formulated in a powder that has a particle size of between about 1000 and about 1500 microns.

12. The composition of claim 11, wherein the powder has a particle size of about 1190 microns.

13. The composition of any one of claims 1-12, further comprising riluzole.

14. The composition of any one of claims 1-12, wherein the composition is for administration in combination with an anti-inflammatory compound.

15. The composition of claim 14, wherein the anti-inflammatory compound is a member of the tetracycline family.

16. The composition of claim 15, wherein the member of the tetracycline family is minocycline.

17. The composition of claim 16, wherein the anti-inflammatory compound is a cyclooxygenase-2 (COX-2) selective inhibitor.

18. The composition of claim 17, wherein the COX-2 selective inhibitor is celecoxib.
